# EUROPEAN PATENT APPLICATION

(11) **EP 4 312 027 A1**
(43) Date of publication of application: **31.01.2024**
(21) Application number: 22759314.2
(22) Date of filing: 02.02.2022
(51) Int. Cl.: G01N 33/58, G01N 33/68, G01N 33/96, G01N 27/62, G01N 30/04, G01N 30/72, C07K 14/47

(54) **QUALITY CONTROL STANDARD SOLUTION USED IN PEPTIDE ASSAY, AND QUALITY CONTROL OF PEPTIDE ASSAY**

(30) Priority: 25.02.2021 JP 2021028347
(71) Applicant: Shimadzu Corporation, Kyoto 604-8511 (JP)
(72) Inventor: YODA, Ritsuko, Kyoto-shi, Kyoto 604-8511 (JP)
(74) Representative: Mewburn Ellis LLP
(86) International application number: PCT/JP2022/004082
(87) International publication number: WO 2022/181273

(57) **Abstract**

A quality control standard for peptide (including protein) measurement, and a quality control for peptide (including proteins) measurement are provided. A QC standard sample for measurement of a target peptide, the QC standard sample comprising: a blood sample comprising a peptide, and a peptide labeled with a stable isotope that is spiked into said blood sample. The target peptide to be measured includes, for example, at least one selected from the group consisting of Aβ1-42 (SEQ ID NO.: 9), Aβ1-38 (SEQ ID NO.: 11), Aβ1-40 (SEQ ID NO.: 7), and APP669-711 (SEQ ID NO.: 10). The peptide labeled with a stable isotope that is spiked into the blood sample includes, for example, at least one selected from the group consisting of SIL-Aβ1-42, SIL-Aβ1-38, SIL-Aβ1-40, and SIL-APP669-711.

## Description

### TECHNICAL FIELD

The present invention pertains to fields of an analysis of a living body-derived sample and of a quantitative analysis of a peptide (including a protein). The present invention relates to a quality control standard solution for peptide measurement, and a quality control for peptide measurement.

### BACKGROUND ART

An analysis of various living body-derived samples and a quantitative analysis of peptides (including proteins) are performed by using an immunoprecipitation (IP) - mass spectrometry (IP-MS), a liquid chromatography - mass spectrometry (LC-MS), a gas chromatography - mass spectrometry (GC-MS), and the like.

When multiple samples of peptides are measured by using such analytical instruments, a quality control of the obtained analytical data is important. That is, in measuring the multiple samples of peptides, it is required to obtain analytical data in a high accuracy based on certain criteria, for any samples, irrespective of the time at which the samples were measured.

For example, Non-Patent Document 1 discloses procedures for large-scale metabolic profiling of serum and plasma using gas chromatography and liquid chromatography coupled to mass spectrometry. The Non-Patent Document 1 discloses that in GC-TOF-MS analysis, QC samples are injected at the start of each analytical batch at item 19 on page 1073. The Non-Patent Document 1 also discloses that in UPLC-TOF-MS analysis, QC samples are injected at the start of each analytical batch at column of BOX 1 on page 1074.

It is conceivable that measurements of a quality control standard solution are carried out before and after measuring consecutive multiple samples, in ordinary measurements, in order to determine whether the obtained analytical data should be accepted or not from the viewpoint of quality.

Meanwhile, in recent years, various peptides (including proteins) useful as biomarkers have been found, and an accurate quantitative analysis thereof is required.

For example, WO 2015/178398 (a family thereof: US 2017/0184573) and WO 2017/047529 (a family thereof: US 2018/0238909) disclose a surrogate biomarker for evaluating intracerebral amyloid β peptide (Aβ) accumulation and a method for analysis thereof.

### CITATION LIST

### PATENT DOCUMENTS

Patent Document 1: WO 2015/178398
Patent Document 2: US 2017/0184573
Patent Document 3: WO 2017/047529
Patent Document 4: US 2018/0238909

### NON-PATENT DOCUMENT

Non-Patent Document 1: NATURE PROTOCOLS, 2011, VOL.6, NO.7, 1060-1083, "Procedures for large-scale metabolic profiling of serum and plasma using gas chromatography and liquid chromatography coupled to mass spectrometry".

### SUMMARY OF THE INVENTION

### PROBLEMS TO BE SOLVED BY THE INVENTION

In measuring the multiple samples of peptides as biomarkers, it is required to obtain analytical data in a high accuracy based on certain criteria, for any samples, irrespective of the time at which the samples were measured. However, each of the above listed Patent Documents fails to make mention of a quality control of the obtained analytical data when the multiple samples of peptides are measured.

Therefore, an object of the present invention is to provide a quality control standard for peptide (including protein) measurement, and a quality control for peptide (including proteins) measurement.

### MEANS FOR SOLVING THE PROBLEMS

In the present description, a quality control standard for peptide measurement may be referred to as a "QC standard sample", a "QC standard solution", or a "QC sample", or may be simply referred to as a "standard sample", a "standard solution", or the like.

In measuring the multiple samples of peptides, it is required to obtain analytical data in a high accuracy based on certain criteria, for any samples, irrespective of the time at which the samples were measured. In order to do that, before measuring an actual sample (i.e., a sample to be measured) and/or after measuring the actual sample (i.e., the sample to be measured), measurements of a quality control standard solution are carried out to verify that a change of the measurement results of the quality control standard solution is not substantially occurred or is small. If the change of the measurement results of the quality control standard solution is not substantially occurred or is small, it is considered that the result of measurement of the actual sample (i.e., the sample to be measured) that was carried out between the measurements of the quality control standard solution was appropriate from the viewpoint of quality.

It is desired that a quality control standard solution is the same as or close to an actual sample (i.e., a sample to be measured) in a composition condition, if possible. From this point of view, in analyses of the multiple samples, it is desired that a small portion of each of the multiple samples is gathered and pooled together to prepare a quality control standard solution. However, there are cases when all the multiple samples have not been collected yet before starting the project analyses of the multiple samples, and cases when there is only a small amount of each of the multiple samples. In such cases, it is difficult that a portion of each of the multiple samples is gathered to prepare a quality control standard solution.

From these circumstances, a commercially available (human) blood sample (including serum sample, and plasma sample) is used as a quality control standard solution.

However, when a commercially available (human) blood sample (including serum sample, and plasma sample) is used as a quality control standard solution, a content of a target peptide to be measured contained in the commercially available (human) blood sample is unknown. There is a possibility that the content of the target peptide to be measured contained in the commercially available (human) blood sample is largely different from a content of said target peptide to be measured contained in an actual sample (i.e., a sample to be measured).

For example, in case that a target biomarker peptide to be measured is an amyloid beta (Aβ) related peptide, it is unknown what content of an amyloid beta (Aβ) related peptide is contained in the commercially available (human) blood sample (including serum sample, and plasma sample). There is a possibility that the content of the amyloid beta (Aβ) related peptide contained in the commercially available (human) blood sample is largely different from a content of said amyloid beta (Aβ) related peptide contained in an actual sample (i.e., a sample to be measured). As such, even if the target biomarker peptide to be measured is spiked (added) into the commercially available (human) blood sample, a suitable spike amount of said target biomarker peptide cannot be determined.

In the first place, the commercially available (human) blood sample is not for the purpose of use in measuring a specific biomarker peptide.

As a result of diligent efforts, the present inventor has found that a quality control for analytical data can be performed by spiking a peptide labeled with a stable isotope into a blood sample to prepare a quality control standard solution, and using an amount of the peptide labeled with a stable isotope (or, in case of two or more kinds of the peptides labeled with a stable isotope, using a ratio of amounts of the respective peptides labeled with a stable isotope) as the basis. The peptide labeled with a stable isotope is not originally existing in a living body-derived blood sample as well as a pseudo blood sample. However, if a basic structure of the peptide labeled with a stable isotope is the same as or analogous to that of a target peptide to be measured, the peptide labeled with a stable isotope can be analyzed due to the same physical and chemical behavior.

The present invention includes the following aspects.

A QC standard sample for measurement of a target peptide, the QC standard sample comprising:
a blood sample comprising a peptide, and
a peptide labeled with a stable isotope that is spiked into said blood sample.

A measurement method of a target peptide in multiple blood samples to be measured, the method comprising the steps of:
a start QC step of performing a measurement of a QC standard sample that comprises a blood sample comprising a peptide, and a peptide labeled with a stable isotope that is spiked into said blood sample;
a sample measurement step of performing a measurement of one or more blood samples to be measured;
an intermediate QC step of performing a measurement of the QC standard sample;
a repeating step of repeating the sample measurement step and the intermediate QC step predetermined number of times;
a sample measurement step of performing a measurement of one or more blood samples to be measured; and
a final QC step of performing a measurement of the QC standard sample.

The measurement method of a peptide according to the above item, wherein in each of the sample measurement steps, consecutive measurements of not less than one and not more than nine blood samples to be measured are carried out.

The measurement method of a peptide according to the above items, wherein the measurement method further comprises the steps of:
a judgement step of deciding that if a measured level of the peptide labeled with a stable isotope in each QC standard sample in the start QC step, the intermediate QC step and the final QC step is within a normal range of not less than minus (-) 20 % and not more than plus (+) 20 % with respect to a predetermined reference value, the measurement of said QC standard sample is accepted, whereas if a measured level of the peptide labeled with a stable isotope in each QC standard sample in the start QC step, the intermediate QC step and the final QC step is out of the normal range, the measurement of said QC standard sample is rejected; and
an adoption determination step of determining that if both measurements of the measurement of the QC standard sample performed at the QC step immediately before each sample measurement step and the measurement of the QC standard sample performed at the QC step immediately after said sample measurement step are accepted, the results of the measurement of one or more blood samples in said sample measurement step are adopted, whereas if either one or both measurements of the measurement of the QC standard sample performed at the QC step immediately before each sample measurement step and the measurement of the QC standard sample performed at the QC step immediately after said sample measurement step are rejected, the results of the measurement of one or more blood samples in said sample measurement step are not adopted.

A QC standard sample kit for measurement of a target peptide, the QC standard sample kit comprising:
a blood sample comprising a peptide, and
a peptide labeled with a stable isotope that is to be spiked into said blood sample.

In the present invention, the term "peptide" includes also "protein".
The term "level of peptide" basically means a concentration, but may be other units applied correspondingly to concentration by a person skilled in the art, for example, may be an ion intensity detected in mass spectrometry.

A target sample to be measured may be a living body-derived sample (for example, a body fluid, such as blood, cerebrospinal fluid (CSF), urine, body secreting fluid, saliva, and sputum; and feces) derived from human, and mammals other than human (for example, rat, dog, cat etc.). The living body-derived sample is disposed of rather than being returned to the test subject (for example, the individual subject) from which the biological sample is derived.

### EFFECTS OF THE INVENTION

According to the present invention, a QC standard sample for measurement of a target peptide comprises a blood sample comprising a peptide, and a peptide labeled with a stable isotope that is spiked into said blood sample. The peptide labeled with a stable isotope is not originally existing in a living body-derived blood sample as well as a pseudo blood sample. Accordingly, a quality control for analytical data can be performed by spiking a peptide labeled with a stable isotope into a blood sample to prepare a QC standard sample, and using an amount of the peptide labeled with a stable isotope as the basis, without being influenced by various fluctuation factors.

According to the present invention, a QC standard sample for measurement of a target peptide comprises a blood sample comprising a peptide, and a peptide labeled with a stable isotope that is spiked into said blood sample. The peptide labeled with a stable isotope is not originally existing in a living body-derived blood sample as well as a pseudo blood sample. However, if a chemical structure of the peptide labeled with a stable isotope is the same as or analogous to that of a target peptide to be measured, the peptide labeled with a stable isotope can be analyzed due to the same physical and chemical behavior. Accordingly, a quality control for analytical data can be performed by spiking a peptide that has the same chemical structure as or an analogous chemical structure to that of a target peptide to be measured and that is labeled with a stable isotope into a blood sample to prepare a QC standard sample, and using an amount of the peptide labeled with a stable isotope as the basis, without being influenced by various fluctuation factors.

In the case where there are two or more kinds of the peptides labeled with a stable isotope, a quality control with a higher degree of reliability for analytical data can be performed by using a ratio of amounts of the respective peptides labeled with a stable isotope as the basis.

According to the present invention, a quality control for analytical data can be performed by using a QC standard sample for measurement of a target peptide, in measuring the target peptide to be measured in the multiple blood samples.

Furthermore, according to the present invention, provided is a kit for preparing the QC standard sample for measurement of a target peptide.

### BRIEF DESCRIPTION OF THE DRAWINGS

[Fig. 1] Fig. 1 shows a schematic measurement flow diagram in measuring multiple samples to be measured.
[Fig. 2] Fig. 2 shows a flow chart for determining a reference value of a ratio of SIL-amyloid peptides in QC sample (standard plasma) from N=27 data.
[Fig. 3] Fig. 3 shows an example of sample position on a 96 well plate in conducting a mass spectrometry analysis of multiple samples to be measured (the number of samples: 42).

### MODES FOR CARRYING OUT THE INVENTION

In an embodiment of the present invention, a QC standard sample for measurement of a target peptide comprises:
a blood sample comprising a peptide, and
a peptide labeled with a stable isotope that is spiked into said blood sample.

[Analyte substances]

First, analyte substances to be measured are described in detail.

The analyte substances to be analyzed are not particularly limited, and examples thereof include peptides, glycopeptides, sugar chains, proteins, lipids, and glycolipids. The peptides, glycopeptides, sugar chains, proteins, lipids, and glycolipids include those of various kinds. More specifically, the analyte substances may be Aβ and an Aβ related peptide. The "Aβ and an Aβ related peptide" may simply collectively be called "Aβ related peptides". The "Aβ and an Aβ related peptide" includes Aβ generated by cleaving amyloid precursor protein (APP) and peptides containing even part of the sequence of Aβ.

For example, Aβ related peptides include the followings.

APP677-709 (Aβ6-38) (SEQ ID NO.: 1):
   HDSGYEVHHQKL VFF AEDVGSNKGAIIGLMVGG
APP672-704 (Aβ1-33) (SEQ ID NO.: 2):
   DAEFRHDSGYEVHHQKLVFFAEDVGSNKGAIIG
APP677-711 (Aβ6-40) (SEQ ID NO.: 3):
   HDSGYEVHHQKL VFF AEDVGSNKGAIIGLMVGGVV
APP672-706 (Aβ1-35) (SEQ ID NO.: 4):
   DAEFRHDSGYEVHHQKLVFFAEDVGSNKGAIIGLM
APP672-708 (Aβ1-37) (SEQ ID NO.: 5):
   DAEFRHDSGYEVHHQKLVFFAEDVGSNKGAIIGLMVG
APP674-711 (Aβ3-40) (SEQ ID NO.: 6):
   EFRHDSGYEVHHQKLVFFAEDVGSNKGAIIGLMVGGVV
APP672-711 (Aβ1-40) (SEQ ID NO.: 7):
   DAEFRHDSGYEVHHQKLVFFAEDVGSNKGAIIGLMVGGVV
OxAPP672-711 (OxAβ1-40) (SEQ ID NO.: 8):
   DAEFRHDSGYEVHHQKLVFFAEDVGSNKGAIIGLMVGGVV (Met 706 is oxidized)
APP672-713 (Aβ1-42) (SEQ ID NO.: 9):
   DAEFRHDSGYEVHHQKLVFFAEDVGSNKGAIIGLMVGGVVIA
APP669-711 (SEQ ID NO.: 10):
   VKMDAEFRHD S GYEVHHQKL VFF AED V GSNKGAIIGLMV GGV V
APP672-709 (Aβ1-38) (SEQ ID NO.: 11):
   DAEFRHDSGYEVHHQKLVFFAEDVGSNKGAIIGLMVGG
APP672-710 (Aβ1-39) (SEQ ID NO.: 12):
   DAEFRHDSGYEVHHQKLVFFAEDVGSNKGAIIGLMVGGV

Amyloid precursor protein (APP) is a single-pass transmembrane protein and is composed of 770 amino acid residues. Amyloid precursor protein (APP) is proteolyzed by β secretase and γ secretase, and an amyloid β peptide (Aβ) is produced by the proteolysis. APP672-713 and Aβ1-42 indicate the same peptide (SEQ ID NO.: 9). APP672-711 and Aβ1-40 indicate the same peptide (SEQ ID NO.: 7).

Among the above-described Aβ related peptides, Aβ1-42 (SEQ ID NO.: 9), Aβ1-40 (SEQ ID NO.: 7), APP669-711 (SEQ ID NO.: 10), and Aβ1-39 (SEQ ID NO.: 12) are effective biomarkers regarding Alzheimer's disease. Furthermore,

A ratio of APP669-711 level to APP672-713 (Aβ1-42) level: APP669-711/APP672-713 (Aβ1-42);
a ratio of APP672-711 (Aβ1-40) level to APP672-713 (Aβ1-42) level: APP672-711 (Aβ1-40)/APP672-713 (Aβ1-42);
a ratio of APP674-711 (Aβ3-40) level to APP672-713 (Aβ1-42) level: APP674-711 (Aβ3-40)/APP672-713 (Aβ1-42), and
a ratio of APP672-710 (Aβ1-39) level to APP672-713 (Aβ1-42) level: APP672-710 (Aβ1-39)/APP672-713 (Aβ1-42) are also examples of effective biomarkers regarding Alzheimer's disease.

Also, the peptides may be those obtained by immunoprecipitation (IP). Alternatively, the peptides may be those generated by digestion of protein with an enzyme such as peptidase, or those fractionated by chromatography.

The analyte substances may include an internal standard substance. The internal standard substance may appropriately be selected by those skilled in the art. For example, a substance labeled with a stable isotope may be used. One substance of the analyte substances may be labeled with a stable isotope. In Examples, examples using stable isotope-labeled Aβ1-38 (SIL-Aβ1-38) as an internal standard substance are shown. In this description, "SIL-" indicates "stable isotope labeled".

A sample containing the analyte substances is subjected to mass spectrometry. The sample to be subjected to mass spectrometry is not particularly limited, and may be, for example, a living body-derived sample. The living body-derived sample includes body fluids such as blood, cerebrospinal fluid (CSF), urine, body secreting fluid, saliva, and sputum; and feces. The blood sample includes a whole blood, a plasma, a serum and the like. The blood sample can be prepared by appropriately treating whole blood collected from an individual. The treatment performed in the case of preparing a blood sample from collected whole blood is not particularly limited, and any treatment that is clinically acceptable may be performed. For example, centrifugal separation or the like may be performed. The blood sample to be subjected to mass spectrometry may be appropriately stored at a low temperature such as freezing in the intermediate stage of the preparation step or in the post stage of the preparation step. In the present invention, when the living body-derived sample such as a blood sample is subjected to mass spectrometry, the living body-derived sample is disposed of rather than being returned to the individual subject from which it is derived.

The sample to be subjected to mass spectrometry may be one that has been subjected to various pretreatments. For example, the sample may be one that has been subjected to immunoprecipitation (IP). The sample may be one that has been subjected to protein digestion with an enzyme such as peptidase. The sample may be one that has been subjected to chromatography. The sample to be subjected to mass spectrometry may be one to which a certain amount of an internal standard substance has been added.

As the sample to be subjected to mass spectrometry, an eluate obtained by immunoprecipitation previously performed may be subjected to mass spectrometry (Immunoprecipitation-mass spectrometry; IP-MS). The immunoprecipitation may be performed using an antibody-immobilized carrier prepared using immunoglobulin having an antigen-binding site that can recognize an analyte substance or an immunoglobulin fragment containing an antigen-binding site that can recognize an analyte substance.

Consecutive immunoprecipitation (cIP) may be conducted on the sample to be subjected to mass spectrometry, and then a peptide in the sample may be detected by a mass spectrometer (cIP-MS). By conducting affinity purification twice consecutively, impurities that cannot be excluded by one affinity purification can be further reduced by the second affinity purification. Therefore, it is possible to prevent the ionization suppression of polypeptide due to impurities, and it becomes possible to measure even a very small amount of polypeptide in a living body sample with high sensitivity by mass spectrometry.

### [QC standard sample]

A QC standard sample for measurement of a target peptide comprises:
a blood sample comprising a peptide, and
a peptide labeled with a stable isotope that is spiked into said blood sample.

In the case where the blood sample for the QC standard sample is a living body-derived sample, various kinds of peptides are usually contained in the blood sample. The peptides contained in the blood sample may include a target peptide(s) to be measured, but the peptides contained in the blood sample may include a peptide(s) that is not a target peptide(s) to be measured. The blood sample may be selected from the group consisting of a whole blood, a plasma, and a serum. The blood sample may have been subjected to a usual pretreatment.

In the case where the blood sample for the QC standard sample is other than a living body-derived sample, the blood sample may be selected from the group consisting of a pseudo blood, a pseudo plasma, and a pseudo serum. In case of the above-mentioned pseudo sample, a desired peptide is preferably spiked (added) into the pseudo sample, according to a target analyte(s) to be measured.

The peptide labeled with a stable isotope that is spiked into the blood sample for the QC standard sample may preferably include a peptide that has the same chemical structure as or an analogous chemical structure to that of the target peptide to be measured and that is labeled with a stable isotope. If the QC standard sample includes a peptide labeled with a stable isotope that has correspondingly the same chemical structure as or an analogous chemical structure to that of the target peptide to be measured, a composition of the QC standard sample may preferably become close to a composition condition of an actual target sample to be measured (i.e., a sample to be measured).

Here, it is described as to a peptide that has an "analogous" chemical structure to that of the target peptide to be measured. For example, in the case where the target peptide to be measured is a peptide selected from amyloid beta (Aβ) related peptides, a peptide included in the amyloid beta (Aβ) related peptides may fall under a peptide that has an "analogous" chemical structure to that of the target peptide to be measured. In these cases, since the QC standard sample includes a peptide labeled with a stable isotope that has correspondingly an "analogous" chemical structure to that of the target peptide to be measured, a composition of the QC standard sample may preferably become close to a composition condition of an actual target sample to be measured (i.e., a sample to be measured).

A peptide that has the same chemical structure as or an analogous chemical structure to that of the target peptide to be measured and that is labeled with a stable isotope is not originally existing in a living body-derived blood sample as well as a pseudo blood sample. However, if a chemical structure of the peptide labeled with a stable isotope is the same as or analogous to that of a target peptide to be measured, the peptide labeled with a stable isotope can be analyzed due to the same physical and chemical behavior. Accordingly, a quality control for analytical data can be performed by spiking a peptide that has the same chemical structure as or an analogous chemical structure to that of a target peptide to be measured and that is labeled with a stable isotope into a blood sample to prepare the QC standard sample, and using an amount of the peptide labeled with a stable isotope as the basis, without being influenced by various fluctuation factors.

If the QC standard sample includes a peptide labeled with a stable isotope that has correspondingly the "same" chemical structure as that of the target peptide to be measured, a composition of the QC standard sample may preferably become more close to a composition condition of an actual target sample to be measured (i.e., a sample to be measured) than the case where the QC standard sample includes a peptide labeled with a stable isotope that has correspondingly an "analogous" chemical structure to that of the target peptide to be measured.

Furthermore, the target peptide to be measured may include multiple kinds of peptides. In this case, the peptide labeled with a stable isotope that is spiked into the blood sample for the QC standard sample may include multiple kinds of peptides that respectively have the same chemical structure as or the analogous chemical structure to that of the multiple kinds of target peptides to be measured and that are labeled with a stable isotope. Since the QC standard sample includes the peptides labeled with a stable isotope that correspond to the target peptides to be measured, a composition of the QC standard sample may preferably become close to a composition condition of an actual target sample to be measured (i.e., a sample to be measured).

The peptide labeled with a stable isotope that is spiked into the blood sample for the QC standard sample may be contained in the QC standard sample in an amount within a predetermined reference range, considering a value close to a composition condition of an actual target sample to be measured (i.e., a sample to be measured). An actual contained amount of the peptide labeled with a stable isotope in the QC standard sample can be a "reference value".

In detail, in the case where the target peptide to be measured is an amyloid beta (Aβ) related peptide, it is preferable that the peptide labeled with a stable isotope that is spiked into the blood sample is an amyloid beta (Aβ) related peptide labeled with a stable isotope. A composition of the QC standard sample may preferably become close to a composition condition of an actual target sample to be measured (i.e., a sample to be measured).

In the case where the target peptide to be measured includes at least one selected from the group consisting of Aβ1-42 (SEQ ID NO.: 9), Aβ1-38 (SEQ ID NO.: 11), Aβ1-40 (SEQ ID NO.: 7), and APP669-711 (SEQ ID NO.: 10), the peptide labeled with a stable isotope that is spiked into the blood sample may include at least one selected from the group consisting of SIL-Aβ1-42, SIL-Aβ1-38, SIL-Aβ1-40, and SIL-APP669-711. Here, "SIL-" indicates "stable isotope labeled".

For example, the stable isotope labeled SIL-Aβ1-38 may be SIL-Aβ1-38 in which carbon atoms in Phe and Ile are substituted by ¹³C, or SIL-Aβ1-38 in which all the nitrogen atoms ¹⁴N are substituted by ¹⁵N.

The stable isotope labeled SIL-Aβ1-42 may be SIL-Aβ1-42 in which carbon atoms in Phe and Ile are substituted by ¹³C, or SIL-Aβ1-42 in which all the nitrogen atoms ¹⁴N are substituted by ¹⁵N.

The stable isotope labeled SIL-Aβ1-40 may be SIL-Aβ1-40 in which carbon atoms in Phe and Ile are substituted by ¹³C, or SIL-Aβ1-40 in which all the nitrogen atoms ¹⁴N are substituted by ¹⁵N.

The stable isotope labeled SIL-APP669-711 may be SII,-APP669-711 in which carbon atoms in Phe and Ile are substituted by ¹³C, or SII,-APP669-711 in which all the nitrogen atoms ¹⁴N are substituted by ¹⁵N.

Thus, the peptide labeled with a stable isotope may be the peptide in which carbon atoms in Phe and Ile are substituted by ¹³C, or the peptide in which all the nitrogen atoms ¹⁴N are substituted by ¹⁵N. These peptides labeled with a stable isotope may be obtained by chemical synthesis, or may be obtained as commercially available product.

Specifically, in the case where the target peptide to be measured includes:
Aβ1-42 (SEQ ID NO.: 9); and
at least one selected from the group consisting of Aβ1-40 (SEQ ID NO.: 7), and APP669-711 (SEQ ID NO.: 10), and
a ratio of measured peptide level:
   a ratio of Aβ1-40/Aβ1-42; and/or
   a ratio of APP669-711/Aβ1-42,
   is/are used as a biomarker;
it is preferable that the peptide labeled with a stable isotope that is spiked into the blood sample includes:
   SIL-Aβ1-42; and
   at least one selected from the group consisting of SIL-Aβ1-40, and SIL-APP669-711, and
   a ratio of abundance of the peptide labeled with a stable isotope:
      a ratio of SIL-Aβ1-40 / SIL-Aβ1-42; and/or
      a ratio of SIL-APP669-711 / SIL-Aβ1-42,
      is/are within a predetermined reference range. A composition of the QC standard sample may preferably become close to a composition condition of an actual target sample to be measured (i.e., a sample to be measured).

More specifically, it is preferable that a ratio of peak intensity in a mass spectrometry analysis as the abundance of the peptide labeled with a stable isotope:
a peak intensity ratio of SIL-Aβ1-40 / SIL-Aβ1-42 is within a predetermined reference range from 25.0 to 32.5; and/or
a peak intensity ratio of SIL-APP669-711 / SIL-Aβ1-42 is within a predetermined reference range from 0.7 to 1.3. A composition of the QC standard sample may preferably become close to a composition condition of an actual target sample to be measured (i.e., a sample to be measured).

A spiked (added) amount of the peptide labeled with a stable isotope may be adjusted so that each peak intensity ratio should be within the above-described range of values.

The peak intensity ratio may be calculated, for example, by standardizing each obtained peak intensity by use of an internal standard peptide (SIL-Aβ1-38), and thereby calculating a peak intensity ratio. Furthermore, the peak intensity ratio may be calculated by obtaining a corrected value of peak intensity by each mass spectrometer, and thereby calculating a peak intensity ratio.

Thus, the peptide labeled with a stable isotope may be the peptide in which carbon atoms in Phe and Ile are substituted by ¹³C, or the peptide in which all the nitrogen atoms ¹⁴N are substituted by ¹⁵N. These peptides labeled with a stable isotope may be obtained by chemical synthesis, or may be obtained as commercially available product.

As to a ratio of peak intensity in a mass spectrometry analysis as the abundance of the peptide labeled with a stable isotope that is spiked into the blood sample for the QC standard sample, an actual contained amount of the peptide labeled with a stable isotope in the QC standard sample can be a "reference value".

As to the abundance of the peptide labeled with a stable isotope that is spiked into the blood sample for the QC standard sample, for example, considering that the abundance of said peptide makes a composition of the QC standard sample close to a composition condition of an actual target sample to be measured (i.e., a sample to be measured), it is preferable that
a concentration of SIL-Aβ1-40 is 45 to 121 pM,
a concentration of SIL-Aβ1-42 is 4 to 13 pM, and
a concentration of SIL-APP669-711 is:4 to 11 pM,
as a concentration in the QC standard sample, respectively. However, these are not restricted to the such concentration ranges. Further, it is useful to use the above-mentioned ratio of peak intensity in a mass spectrometry analysis as the reference, since the ratio of actual abundance (pM) and the ratio of peak intensity in a mass spectrometry analysis do not necessarily correspond to each other. The ratio of actual peptide concentration and the ratio of peak intensity in a mass spectrometry analysis may be different from each other, since an immunoprecipitation (IP) yield and an ionization efficiency in a mass spectrometry (MS) vary depending on type of peptides.

### [Peptide measurement method]

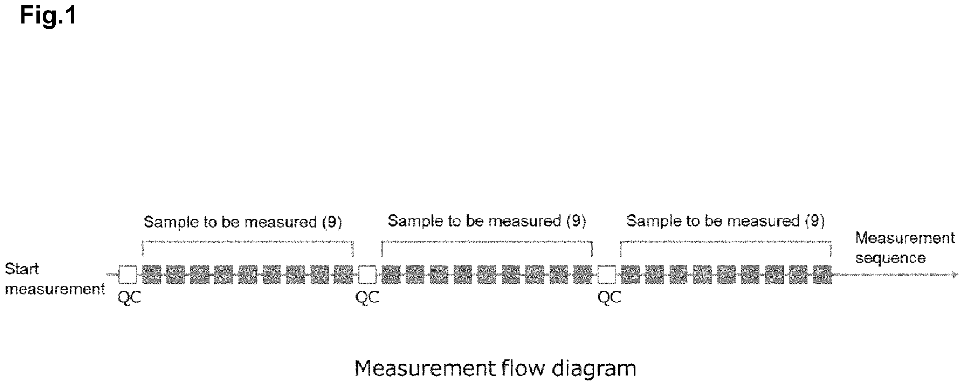

In an embodiment of the present invention, referring to Fig. 1, a measurement method of a target peptide in multiple blood samples to be measured comprises the steps of:
a start QC step of performing a measurement of a QC standard sample that comprises a blood sample comprising a peptide, and a peptide labeled with a stable isotope that is spiked into said blood sample;
a sample measurement step of performing a measurement of one or more blood samples to be measured;
an intermediate QC step of performing a measurement of the QC standard sample;
a repeating step of repeating the sample measurement step and the intermediate QC step predetermined number of times;
a sample measurement step of performing a measurement of one or more blood samples to be measured; and
a final QC step of performing a measurement of the QC standard sample.

In each of the sample measurement steps, consecutive measurements of the blood samples to be measured are carried out. For example, consecutive measurements of not less than one and not more than nine blood samples to be measured may be carried out.

In an embodiment of the present invention, the measurement method of a target peptide in multiple blood samples to be measured may further comprises the steps of:
a judgement step of deciding that if a measured level of the peptide labeled with a stable isotope in each QC standard sample in the start QC step, the intermediate QC step and the final QC step is within a normal range of not less than minus (-) 20 % and not more than plus (+) 20 % with respect to a predetermined reference value, the measurement of said QC standard sample is accepted, whereas if a measured level of the peptide labeled with a stable isotope in each QC standard sample in the start QC step, the intermediate QC step and the final QC step is out of the normal range, the measurement of said QC standard sample is rejected; and
an adoption determination step of determining that if both measurements of the measurement of the QC standard sample performed at the QC step immediately before each sample measurement step and the measurement of the QC standard sample performed at the QC step immediately after said sample measurement step are accepted, the results of the measurement of one or more blood samples in said sample measurement step are adopted, whereas if either one or both measurements of the measurement of the QC standard sample performed at the QC step immediately before each sample measurement step and the measurement of the QC standard sample performed at the QC step immediately after said sample measurement step are rejected, the results of the measurement of one or more blood samples in said sample measurement step are not adopted.

The target peptide to be measured may be an amyloid beta (Aβ) related peptide.

The peptide labeled with a stable isotope contained in the QC standard sample may be an amyloid beta (Aβ) related peptide that is labeled with a stable isotope.

In the case where the target peptide to be measured includes at least one selected from the group consisting of Aβ1-42 (SEQ ID NO.: 9), Aβ1-38 (SEQ ID NO.: 11), Aβ1-40 (SEQ ID NO.: 7), and APP669-711 (SEQ ID NO.: 10), the peptide labeled with a stable isotope contained in the QC standard sample may include at least one selected from the group consisting of SIL-Aβ1-42, SIL-Aβ1-38, SIL-Aβ1-40, and SIL-APP669-711. Here, "SIL-" indicates "stable isotope labeled".

Specifically, it is preferable that the target peptide to be measured includes:
Aβ1-42 (SEQ ID NO.: 9); and
at least one selected from the group consisting of Aβ1-40 (SEQ ID NO.: 7), and APP669-711 (SEQ ID NO.: 10), and
a ratio of measured peptide level:
   a ratio of Aβ1-40/Aβ1-42; and/or
   a ratio of APP669-711/Aβ1-42,
   is/are used as a biomarker;
the peptide labeled with a stable isotope contained in the QC standard sample includes:
   SIL-Aβ1-42; and
   at least one selected from the group consisting of SIL-Aβ1-40, and SIL-APP669-711, and
   in the QC standard sample, a ratio of abundance of the peptide labeled with a stable isotope:
      a ratio of SIL-Aβ1-40 / SIL-Aβ1-42; and/or
      a ratio of SIL-APP669-711 / SIL-Aβ1-42,
is/are within a predetermined reference range. A composition of the QC standard sample may preferably become close to a composition condition of an actual target sample to be measured (i.e., a sample to be measured).

More specifically, it is preferable that, in the QC standard sample, a ratio of peak intensity in a mass spectrometry analysis as the abundance of the peptide labeled with a stable isotope:
a peak intensity ratio of SIL-Aβ1-40 / SIL-Aβ1-42 is within a reference range from 25.0 to 32.5; and/or
a peak intensity ratio of SIL-APP669-711 / SIL-Aβ1-42 is within a reference range from 0.7 to 1.3. A composition of the QC standard sample may preferably become close to a composition condition of an actual target sample to be measured (i.e., a sample to be measured).

Each ratio of peak intensity in a mass spectrometry analysis for the SIL-peptides labeled with a stable isotope is not restricted to each of the above-mentioned ranges. As to each ratio value, an optimal range value of each said ratio value may varies depending on method of mass spectrometer (MALDI, ESI, or the like), and type of mass spectrometer (manufacturing maker thereof, model type, type of LASER used, and the like). The reason is that the ratio of actual peptide concentration and the ratio of peak intensity in a mass spectrometry analysis may be different from each other, since an immunoprecipitation (IP) yield and an ionization efficiency in a mass spectrometry (MS) vary depending on type of peptides. Each of the above-mentioned ranges is an example in case that measurement was carried out by use of AXIMA-Performance ^{™} manufactured by SHIMADZU.

As to a ratio of peak intensity in a mass spectrometry analysis as the abundance of the peptide labeled with a stable isotope that is spiked into the blood sample for the QC standard sample, a ratio of peptide peak intensity that is obtained by actually performing a mass spectrometry analysis of a QC standard plasma can be a "reference value".

A measurement of a peptide can be performed by using an immunoprecipitation (IP) - mass spectrometry (IP-MS), a liquid chromatography - mass spectrometry (LC-MS), or a gas chromatography - mass spectrometry (GC-MS).

### [Mass spectrometry]

The mass spectrometry is not particularly limited, and examples thereof include those for mass spectrometry such as matrix-assisted laser desorption/ionization (MALDI) mass spectrometry or electrospray ionization (ESI) mass spectrometry. For example, a MALDI-TOF (matrix-assisted laser desorption/ionization time-of-flight) mass spectrometer, a MALDI-IT (matrix-assisted laser desorption/ionization ion trap) mass spectrometer, a MALDI-IT-TOF (matrix-assisted laser desorption/ionization ion trap time-of-flight) mass spectrometer, a MALDI-FTICR (matrix-assisted laser desorption/ionization Fourier transform ion cyclotron resonance) mass spectrometer, an ESI-QqQ (electrospray ionization triple quadrupole) mass spectrometer, an ESI-Qq-TOF (electrospray ionization tandem quadrupole time-of-flight) mass spectrometer, or an ESI-FTICR (electrospray ionization Fourier transform ion cyclotron resonance) mass spectrometer or the like can be used.

A matrix and a matrix solvent can be appropriately determined by a person skilled in the art depending on the analyte substance to be measured.

As the matrix, for example, α-cyano-4-hydroxycinnamic acid (CHCA), 2,5-dihydroxybenzoic acid (2,5-DHB), sinapic acid, 3-aminoquinoline (3-AQ) or the like can be used.

The matrix solvent can be selected from the group consisting of, for example, acetonitrile (ACN), trifluoroacetic acid (TFA), methanol, ethanol and water, and used. More specifically, an ACN-TFA aqueous solution, an ACN aqueous solution, methanol-TFA aqueous solution, a methanol aqueous solution, an ethanol-TFA aqueous solution, an ethanol solution or the like can be used. The concentration of ACN in the ACN-TFA aqueous solution can be, for example, 10 to 90% by volume, the concentration of TFA can be, for example, 0.05 to 1% by volume, preferably 0.05 to 0.1% by volume.

The matrix concentration can be, for example, 0.1 to 50 mg/mL, preferably 0.1 to 20 mg/mL, or 0.3 to 20 mg/mL, further preferably 0.5 to 10 mg/mL.

In the case of employing MALDI mass spectrometry as a detecting system, a matrix additive (comatrix) is preferably used together. The matrix additive can be appropriately selected by a person skilled in the art depending on the analysis subject (poly peptides) and/or the matrix. For example, as the matrix additive, a phosphonic acid group-containing compound can be used. Specific examples of a compound containing one phosphonic acid group include phosphonic acid, methylphosphonic acid, phenylphosphonic acid, 1-naphthylmethylphosphonic acid, and the like. Specific examples of a compound containing two or more phosphonic acid groups include methylenediphosphonic acid (MDPNA), ethylenediphosphonic acid, ethane-1-hydroxy-1,1-diphosphonic acid, nitrilotriphosphonic acid, ethylenediaminetetraphosphonic acid, and the like. Among the aforementioned phosphonic acid group-containing compounds, compounds having two or more, preferably two to four phosphonic acid groups in one molecule are preferred.

The use of the phosphonic acid group-containing compound is useful, for example, when metal ions of the washing solution remaining on the surface of the antibody-immobilizing carrier are contaminated into the eluate after the dissociating step. The metal ions adversely affect on the background in the mass spectrometry. The use of the phosphonic acid group-containing compound is effective for suppressing such an adverse affect.

Besides the aforementioned matrix additive, a more common additive, for example, a substance that is selected from the group consisting of ammonium salts and organic bases may be used.

The matrix additive can be prepared as a solution of 0.1 to 10 w/v %, preferably 0.2 to 4 w/v % in water or in a matrix solvent. The matrix additive solution and the matrix solution can be mixed in a volume ratio of, for example, 1 : 100 to 100 : 1, preferably 1 : 10 to 10 : 1.

### [QC standard sample kit]

In an embodiment of the present invention, a QC standard sample kit for measurement of a target peptide comprises:
a blood sample comprising a peptide, and
a peptide labeled with a stable isotope that is to be spiked into said blood sample.

In the QC standard sample kit, the peptide labeled with a stable isotope may include:
SIL-Aβ1-42; and
at least one selected from the group consisting of SIL-Aβ1-40, and SIL-APP669-711. Here, "SIL-" indicates "stable isotope labeled". The QC standard sample kit can also include other required components. Examples of the other required components include SIL-Aβ1-38 which is used as an internal standard, various buffer solutions.

### EXAMPLES

Hereinafter, the present invention will be described specifically with reference to examples, but is not limited to these examples. In the following, the amount of a matter indicated by % is based on volume when the matter is liquid unless otherwise indicated.

### [Experimental Example 1: Preparation of standard plasma]

A protocol example for a preparation method of a QC sample (standard plasma) is shown below.

As SIL-Aβ1-38, a product of AnaSpec (San Jose, CA, USA) was used. In the SIL-Aβ1-38, carbon atoms in Phe and Ile of Aβ1-38 are substituted by ¹³C.

As SIL-Aβ1-40, a product of rPeptide (Watkinsville, GA, USA) was used. In the SIL-Aβ1-40, all the nitrogen atoms of Aβ1-40 are substituted by ¹⁵N.

As SIL-Aβ1-42, a product of rPeptide (Watkinsville, GA, USA) was used. In the SIL-Aβ1-42, all the nitrogen atoms of Aβ1-42 are substituted by ¹⁵N.

As SIL-APP669-711, a product of rPeptide (Watkinsville, GA, USA) was used. In the SIL-APP669-711, all the nitrogen atoms of APP669-711 are substituted by ¹⁵N.

### [Experimental Example 1-1: SIL-peptide spike into plasma]

[1] Each of SIL-Aβ1-40, SIL-Aβ1-42, and SIL-APP669-711 was diluted with 1 mg/mL BSA, 0.1 % (w/v) DDM, and 50 mM Tris-HCl buffer (pH 9.0) to prepare 50 nM of SIL-Aβ1-40, 5 nM of SIL-Aβ1-42, and 5 nM of SIL-APP669-711. Each 100 µL of 50 nM of SIL-Aβ1-40, 5 nM of SII,-A(31-42, and 5 nM of SIL-APP669-711 was added to 100 mL of human plasma. The plasma is referred to as "SIL spiked plasma -1".
[2] The SIL spiked plasma -1 was fully mixed by inversion mixing.
[3] 900 µL of the SIL spiked plasma -1 was divided into each of two 1.5 mL volume microtubes. The obtained two microtubes and the remaining SIL spiked plasma -1 were frozen at minus (-) 60 °C or less, respectively.
[4] One of the two microtubes that contains 900 µL of the SIL spiked plasma -1 in the frozen state was melted. And then, a consecutive immunoprecipitation (cIP) - mass spectrometry (cIP-MS) was performed, wherein the IP repeating number of times was three times. The detailed operation of IP-MS is described below.

### [Consecutive Immunoprecipitation (cIP)]

### (Preparation of antibody-immobilizing beads)

Clone 6E10 (available from Covance) of an anti-Aβ antibody (IgG) recognizing 3-8 residues of amyloid β protein (Aβ) as an epitope was prepared.

For 100 µg of an anti-Aβ antibody (IgG), about 3.3 × 10⁸ magnetic beads (Dynabeads (registered trademark) M-270 Epoxy) were reacted in an immobilizing buffer (0.1 M phosphate buffer containing 1.3 M ammonium sulfate (pH 7.4)) at 37 °C for 16 to 24 hours, to prepare anti-Aβ IgG immobilizing beads.

### (First reaction step)

Into 250 µL of the above-mentioned SIL spiked plasma -1, 250 µL of a first IP reaction buffer (0.2 % (w/v) DDM, 0.2 % (w/v) NTM, 800 mM GlcNAc, 100 mM Tris-HCl (pH 7.4), 300 mM NaCl) containing 11 pM stable isotope labeled Aβ1-38 (SIL-Aβ1-38) was mixed, and then the mixture was allowed to stand for 5 to 60 minutes on ice. SIL-Aβ1-38 in which carbon atoms in Phe and Ile are substituted by ¹³C was used as an internal standard for standardization of signal intensity of a mass spectrum. The plasma was mixed with anti-Aβ IgG immobilizing beads, and the resulting mixture was shaken by use of Peltier chiller for 1 hour at 4 °C and pippeting-stirred.

### (First washing step, First elution step)

Then, the antibody beads were washed one time with 100 µL of a first IP washing buffer (0.1 % DDM, 0.1 % NTM, 50 mM Tris-HCl (pH 7.4), 150 mM NaCl), and washed one time with 50 µL of a 50 mM ammonium acetate buffer, and then Aβ and Aβ-like peptides (namely, APP-derived peptides) bound to the antibody beads were eluted with a first IP eluent (50 mM Glycine buffer containing 0.1 % DDM (pH 2.8)).

### (Neutralization step)

The obtained eluate was mixed with a second IP reaction buffer (0.2 % (w/v) DDM, 800 mM GlcNAc, 300 mM Tris-HCl (pH 7.4), 300 mM NaCl) to obtain a first purified solution.

### (Second reaction step)

The obtained first purified solution was mixed with another anti-Aβ antibody immobilizing beads, and the resulting mixture was shaken by use of Peltier chiller for 1 hour at 4 °C and pippeting-stirred.

### (Second washing step, Second elution step)

Then, the anti-Aβ antibody immobilizing beads were washed two times with 50 µL of a second washing buffer (0.1 % DDM, 150 mM Tris-HCl (pH 7.4), 150 mM NaCl), washed one time with 50 µL of a 50 mM ammonium acetate buffer, and washed one time with 30 µL of H₂O, and then Aβ and Aβ-like peptides (APP-derived peptides) bound to the antibody beads were eluted with 8 µL (or, 9 µL, depending on the humidity of the experimental room) of a second IP eluent (70 % (v/v) acetonitrile containing 5 mM hydrochloric acid). In this manner, a second purified solution was obtained. The second purified solution was subjected to a mass spectrometry.
n-Dodecyl-β-D-maltoside (DDM) [critical micelle concentration (cmc): 0.009 %]
n-Nonyl-β-D-thiomaltoside (NTM) [cmc: 0.116 %]

### [Detection by MALDI-TOF MS]

As a matrix for Linear TOF, α-cyano-4-hydroxycinnamic acid (CHCA) was used. A matrix solution was prepared by dissolving 1 mg of CHCA in 1 mL of 70 % (v/v) acetonitrile. As a matrix additive, 0.4 %(w/v) methanediphosphonic acid (MDPNA) was used. After mixing equivalent amounts of a 1 mg/mL CHCA solution and 0.4 % (w/v) MDPNA, 0.5 µL of the resultant mixture was dropped on a µFocus MALDI plate^{™} 900 µm (Hudson Surface Technology, Inc., Fort Lee, NJ) and dried and solidified.

One µL of the second purified solution obtained by the above-mentioned immunoprecipitation was dropped into the matrix on the µFocus MALDI plate^{™} 900 µm.

Mass spectrum data was acquired by Linear TOF in a positive ion mode by using AXIMA Performance (Shimadzu/KRATOS, Manchester, UK). For 1 well, 400 spots, or 16,000 shots were integrated. A criterion of a detection limit of a peak was an S/N ratio of 3 or more. An m/z value of Linear TOF was indicted by an average mass of a peak. An m/z value was calibrated by using human angiotensin II and human ACTH fragment 18-39, bovine insulin oxidized beta-chain, and bovine insulin as external standards.

[5] SIL amyloid peptide ratio (SIL-Aβ1-40 / SIL-Aβ1-42, and SIL-APP669-711 / SIL-Aβ1-42, average value of N=3) was calculated. At this stage, in the case that data cannot be acquired due to a performance degradation of the device, unexpected troubles, or the like, the other one of the two microtubes that contains 900 µL of the SIL spiked plasma -1 in the frozen state can be used to perform again cIP-MS.

[6] In the case where the calculated value was within a reference range, namely,
a ratio of SIL-Aβ1-40 / SIL-Aβ1-42 is from 25.0 to 32.5; and
a ratio of SIL-APP669-711 / SIL-Aβ1-42 is from 0.7 to 1.3, experimental operation was moved on to the item of [Experimental Example 2: Determination of SIL amyloid peptide ratio reference value]. On the other hand, in the case where the calculated value was out of a reference range, experimental operation was moved on to the next described item of [Experimental Example 1-2: Adjustment of SIL amyloid peptide ratio].

### [Experimental Example 1-2: Adjustment of SIL-amyloid peptide ratio].

[1] The remaining SIL spiked plasma -1 that is maintained in the frozen state at [3] in the previous item of Experimental Example 1-1 was defrosted by allowing it to stand at a room temperature to obtain a defrosted solution.
[2] If the other one of the two microtubes that contains 900 µL of the SIL spiked plasma -1 in the frozen state prepared at [3] in the previous item of Experimental Example 1-1 is remained, the other one was defrosted to obtain a defrosted solution, and then, the obtained defrosted solution was mixed into the resulting defrosted solution obtained in the above item [1].
[3] A target value of the SIL amyloid peptide ratio was set as follows:
   a ratio of SIL-Aβ1-40 / SIL-Aβ1-42 is 28.0; and
   a ratio of SIL-APP669-711 / SIL-Aβ1-42 is 1.0.
   Each required amount of 50 nM of SIL-Aβ1-40, 5 nM of SIL-Aβ1-42,and 5 nM of SIL-APP669-711 was added to the resulting defrosted solution obtained in the above item [1], with aiming for an intermediate point (i.e., an intermediate target value) that is an intermediate value between the calculated measured value at [5] in the previous item of Experimental Example 1-1 and the target value. Here, it should be noted that if each SIL peptide is added to the resulting defrosted solution obtained in the above item [1] with aiming for the final target value, each addition amount is often too excess. Therefore, each SIL peptide was added intentionally in an amount with aiming for the intermediate value.
[4] The resulting solution obtained at [3] was fully mixed by inversion mixing. The solution is referred to as "SIL spiked plasma -2".
[5] 900 µL of the SIL spiked plasma -2 was divided into each of two 1.5 mL volume microtubes. The obtained two microtubes and the remaining SIL spiked plasma -2 were frozen at minus (-) 60 °C or less, respectively.
[6] One of the two microtubes that contains 900 µL of the SIL spiked plasma -2 in the frozen state was melted. And then, a consecutive immunoprecipitation (cIP) - mass spectrometry (cIP-MS) was performed, wherein the IP repeating number of times was three times. The detailed operation of IP-MS was as the same manner as in the previous item of Experimental Example 1-1.
[7] SIL-amyloid peptide ratio (SIL-Aβ1-40 / SIL-Aβ1-42, and SIL-APP669-711 / SIL-Aβ1-42, average value of N=3) was calculated. At this stage, in the case that data cannot be acquired due to a performance degradation of the device, unexpected troubles, or the like, the other one of the two microtubes that contains 900 µL of the SIL spiked plasma -2 in the frozen state can be used to perform again cIP-MS.
[8] In the case where the calculated value was within a reference range, namely,
   a ratio of SIL-Aβ1-40 / SIL-Aβ1-42 is from 25.0 to 32.5; and
   a ratio of SIL-APP669-711 / SIL-Aβ1-42 is from 0.7 to 1.3,
   experimental operation was moved on to the item of [Experimental Example 2: Determination of SIL amyloid peptide ratio reference value]. On the other hand, in the case where the calculated value was out of a reference range, experimental operation was moved on to the next described item [9] and after to the third time's spike operation.
[9] The remaining SIL spiked plasma -2 that is maintained in the frozen state at the above item [5] was defrosted by allowing it to stand at a room temperature to obtain a defrosted solution.
[10] If the other one of the two microtubes that contains 900 µL of the SIL spiked plasma -2 in the frozen state prepared at the above item [5] is remained, the other one was defrosted to obtain a defrosted solution, and then, the obtained defrosted solution was mixed into the resulting defrosted solution obtained in the above item [9].
[11] As the third time's spike operation, a target value of the SIL-amyloid peptide ratio was set as follows:
   a ratio of SIL-Aβ1-40 / SIL-Aβ1-42 is 28.0; and
   a ratio of SIL-APP669-711 / SIL-Aβ1-42 is 1.0.
   Each required amount of 50 nM of SIL-Aβ1-40, 5 nM of SIL-Aβ1-42, and 5 nM of SIL-APP669-711 was added to the resulting defrosted solution obtained in the above item [9], with aiming for the target value.
[12] The resulting solution obtained at [11] was fully mixed by inversion mixing. The solution is referred to as "SIL spiked plasma -3".
[13] 900 µL of the SIL spiked plasma -3 was divided into each of two 1.5 mL volume microtubes. The obtained two microtubes and the remaining SIL spiked plasma -3 were frozen at minus (-) 60 °C or less, respectively.
[14] One of the two microtubes that contains 900 µL of the SIL spiked plasma -3 in the frozen state was melted. And then, a consecutive immunoprecipitation (cIP) - mass spectrometry (cIP-MS) was performed, wherein the IP repeating number of times was three times. The detailed operation of IP-MS was as the same manner as in the previous item of Experimental Example 1-1.
[15] SIL-amyloid peptide ratio (SIL-Aβ1-40 / SIL-Aβ1-42, and SIL-APP669-711 / SIL-Aβ1-42, average value of N=3) was calculated. At this stage, in the case that data cannot be acquired due to a performance degradation of the device, unexpected troubles, or the like, the other one of the two microtubes that contains 900 µL of the SIL spiked plasma -3 in the frozen state can be used to perform again cIP-MS.
[16] In the case where the calculated value was within a reference range, namely,
   a ratio of SIL-Aβ1-40 / SIL-Aβ1-42 is from 25.0 to 32.5; and
   a ratio of SIL-APP669-711 / SIL-Aβ1-42 is from 0.7 to 1.3,
   experimental operation was moved on to the item of [Experimental Example 2: Determination of SIL amyloid peptide ratio reference value]. On the other hand, in the case where the calculated value was out of a reference range, returning to the above item [9], a further spike operation and subsequent IP-MS operation were repeated until the calculated value was within the reference range in accordance with the procedures of the above items [9] to [16].

### [Experimental Example 2: Determination of SIL-amyloid peptide ratio reference value in standard plasma].

[1] As a standard plasma, a SIL spiked plasma was used, wherein the SIL-amyloid peptide ratio thereof is within the reference range, namely,
   the ratio of SIL-Aβ1-40 / SIL-Aβ1-42 is from 25.0 to 32.5; and
   the ratio of SIL-APP669-711 / SIL-Aβ1-42 is from 0.7 to 1.3.
[2] The SIL spiked plasma that is maintained in the frozen state was defrosted by allowing it to stand at a room temperature to obtain a defrosted solution.
[3] If the other one of the two microtubes that contains 900 µL of the SIL spiked plasma in the frozen state is remained, the other one was defrosted to obtain a defrosted solution, and then, the obtained defrosted solution was mixed into the resulting defrosted solution obtained in the above item [2].
[4] The whole amount of the defrosted solution was dispensed into each of 1.5 mL volume microtubes with each amount of 900 µL.
[5] All the dispensed products were once frozen at minus (-) 60 °C or less.
[6] Three microtubes of the dispensed products in the frozen state at the above item [5] were melted. And then, the IP-MSs were performed by use of 3 lots of the antibody beads by each N=3 (Total N=9). The detailed operation of IP-MS was as the same manner as in the previous item of Experimental Example 1-1.
[7] The IP-MS operations were repeated for 3 batches (Total N=27).
[8] From the IP-MS results of N=27, the amyloid peptide ratio was calculated. In the case where both of the calculated amyloid peptide ratio values were within the reference range, namely,
   the ratio of SIL-Aβ1-40 / SIL-Aβ1-42 is from 25.0 to 32.5; and
   the ratio of SIL-APP669-711 / SIL-Aβ1-42 is from 0.7 to 1.3,
   the obtained values were determined as the "reference value". In the case where either one or both of the calculated amyloid peptide ratio values were out of the reference range, operations from the preparation of standard plasma were performed again.

According to the above-mentioned protocol example, the QC sample (standard plasma) was prepared.

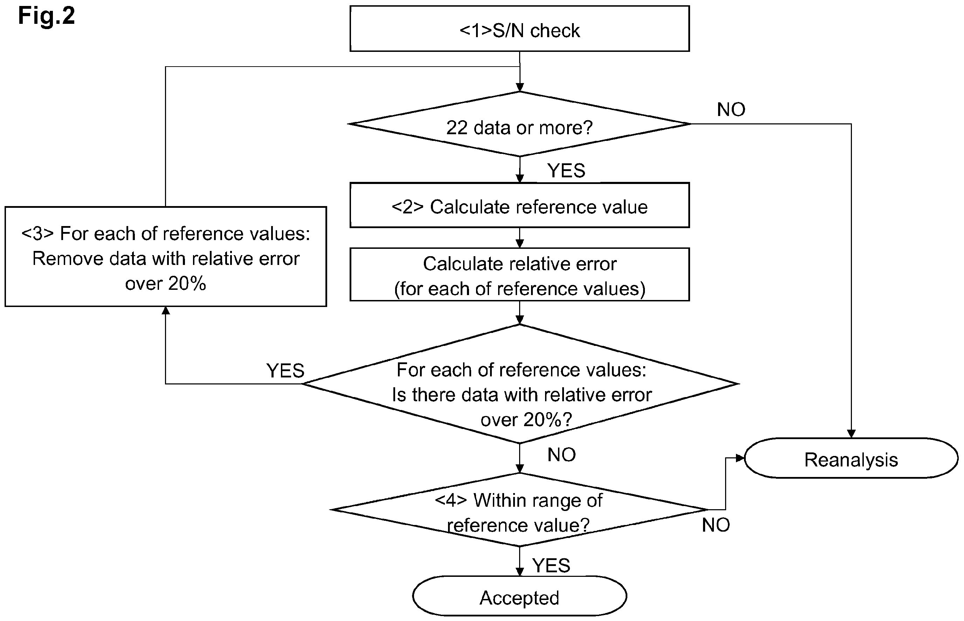

Fig. 2 shows a flow chart for determining a reference value of a ratio of SIL-amyloid peptides in QC sample (standard plasma) from N=27 data.

In Fig. 2,
<1> Concerning the S/N threshold value:
   S/N threshold value of SIL-Aβ1-38: 15,
   S/N threshold value of SII,-Aβ 1-40: 3,
   S/N threshold value of SII,-Aβ 1-42: 3, and
   S/N threshold value of SIL-APP669-711: 3.
<2> Concerning the reference value:
   the reference value 1:
      the ratio of SIL-Aβ1-40 / SIL-Aβ1-42, and
   the reference value 2:
      the ratio of SIL-APP669-711 / SIL-Aβ1-42.
<3> Concerning the removal method:
   Regarding data having relative error over 20 % with respect to the reference value, the largest relative error datum of the data is removed in a sequential order, and recalculation is carried out.
   The data are dealt with independently for each of the reference values. That is, the data are removed as to the reference value 1 since said data have relative error over 20 % with respect to the reference value 1, whereas said data are adopted as to the reference value 2 since said data have relative error of 20 % or less with respect to the reference value 2.

More detailed explanation is described below.

In the determination stage of the reference value, an abnormal value of the data is removed in a sequential order, considering a measurement error. If 22 data or more are finally remained, wherein 22 data or more correspond to 80 % or more with respect to total N=27 data, the average value of the remaining data is calculated. The average value is determined as the reference value. For example, in a certain spectrum, when the peak intensity of SIL-Aβ1-40 is abnormal due to the fact that a peak of impurities overlaps with the peak position of SIL-Aβ1-40, the data are removed as to the reference value 1 (SIL-Aβ1-40 / SIL-Aβ1-42) are removed. On the other hand, the reference value 2 (SII,-APP669-711 / SIL-Aβ1-42.) is adopted since peaks of SIL-APP669-711 and SIL-Aβ1-42 are correctly detected.

Determination of the reference value 1 and the reference value 2 from the measurement data of N=27 can be executed by automatic calculation software.

<4> Concerning the reference value range that is accepted:
the reference value 1: 25.0 to 32.5; and
the reference value 2: 0.7 to 1.3.

### [Experimental Example 3: Measurement of amyloid peptide sample using QC sample (standard plasma)]

Here, amyloid peptide blood samples to be measured (the number of samples: 42) were subjected to IP-MS measurement using the QC sample (standard plasma) prepared according to the above-mentioned protocol example.

### (Fundamental operation of IP-MS measurement)

Each sample to be measured contains SIL-Aβ1-38 (AnaSpec, San Jose, CA, USA) that has been added therein as an internal standard, and has been subjected to IP operation according to the consecutive immunoprecipitation as described in the previous item of Experimental Example 1-1.

MS measurement was performed by MALDI-TOF MS analysis.

In more detail, mass spectrum data was acquired by Linear TOF in a positive ion mode by using AXIMA Performance (Shimadzu/KRATOS, Manchester, UK).

As a matrix for Linear TOF, α-cyano-4-hydroxycinnamic acid (CHCA) was used. A matrix solution was prepared by dissolving 1 mg of CHCA in 1 mL of 70 % (v/v) acetonitrile. As a matrix additive, 0.4 %(w/v) methanediphosphonic acid (MDPNA) was used. After mixing equivalent amounts of a 1 mg/mL CHCA solution and 0.4 % (w/v) MDPNA, 0.5 µL of the resultant mixture was dropped on a µFocus MALDI plate^{™} 900 µm (Hudson Surface Technology, Inc., Fort Lee, NJ) and dried and solidified.

One µL of the purified solution of the sample obtained by the above-mentioned IP operation was dropped into the matrix on the µFocus MALDI plate^{™} 900 µm.

For 1 well, 400 spots, or 16,000 shots were integrated. A criterion of a detection limit of a peak was an S/N ratio of 3 or more. An m/z value of Linear TOF was indicted by an average mass of a peak. An m/z value was calibrated by using human angiotensin II and human ACTH fragment 18-39, bovine insulin oxidized beta-chain, and bovine insulin as external standards.

Referring Fig. 1, as a process example in case that 42 samples of the maximum sample number were processed.

First, a MS measurement of the QC standard sample was performed (the start QC step).

Next, a MS measurement of the each sample to be measured (the number of samples: 9) after subjected to IP was performed (the sample measurement step).

Subsequently, a MS measurement of the QC standard sample was performed (the intermediate QC step).

Next, the sample measurement step and the intermediate QC step were repeated predetermined number of times (three times) (the repeating step)

Next, a MS measurement of the each sample to be measured (the number of samples: 6) after subjected to IP was performed (the sample measurement step).

Subsequently, a MS measurement of the QC standard sample was performed (the final QC step).

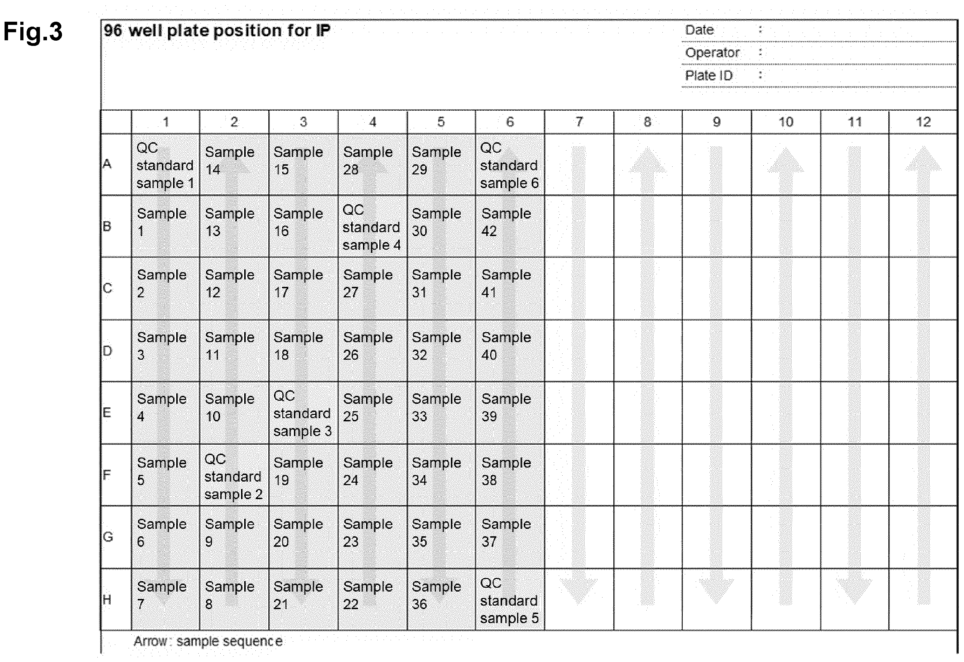

Fig. 3 shows an example of sample position on a 96 well plate in conducting a mass spectrometry analysis of multiple samples to be measured (the number of samples: 42). The sample measurement sequence is indicated as the arrow (→).

Referring Fig. 3, first, the QC standard sample 1 is placed on the plate well 1A (the start QC step),
next, the each sample 1 to 9 is placed on the well 1B to 2G (the sample measurement step), the QC standard sample 2 is placed on the well 2F (the intermediate QC step),
the each sample 11 to 19 is placed on the well 2E to 3F (the sample measurement step), the QC standard sample 3 is placed on the well 3E (the intermediate QC step),
the each sample 18 to 27 is placed on the well 3D to 4C (the sample measurement step), the QC standard sample 4 is placed on the well 4B (the intermediate QC step),
the each sample 28 to 29 is placed on the well 4A to 5A (the sample measurement step), the QC standard sample 5 is placed on the well 6H (the intermediate QC step),
the each sample 37 to 42 is placed on the well 6G to 6B (the sample measurement step), and finally, the QC standard sample 6 is placed on the plate well 6A (the final QC step).

(Quality control of measurement of amyloid peptide sample using QC sample) A level ratio of the peptide labeled with a stable isotope in the each QC standard sample that was used in the start QC step, the intermediate QC step, and the final QC step meets the following requirements:
Regarding the reference value 1:
   the ratio of SIL-Aβ1-40 / SIL-Aβ1-42 is within a range from 25.0 to 32.5; and
Regarding the reference value 2:
   the ratio of SII,-APP669-711 / SIL-Aβ1-42 is within a range from 0.7 to 1.3.

If a ratio of an actually measured level of the peptide labeled with a stable isotope in each QC standard sample in the start QC step, the intermediate QC step and the final QC step was within a normal range of not less than minus (-) 20 % and not more than plus (+) 20 % with respect to the reference value 1 and the reference value 2, said QC standard sample was accepted, whereas if a ratio of an actually measured level of the peptide labeled with a stable isotope in each QC standard sample in the start QC step, the intermediate QC step and the final QC step was out of the normal range, said QC standard sample was rejected (the judgement step).

If both the QC standard sample used at the QC step immediately before each sample measurement step and the QC standard sample used at the QC step immediately after said sample measurement step were accepted, the results of the measurement obtained in said sample measurement step were adopted. On the other hand, if either one or both the QC standard sample used at the QC step immediately before each sample measurement step and the QC standard sample used at the QC step immediately after said sample measurement step were rejected, the results of the measurement obtained in said sample measurement step were not adopted (the adoption determination step).

Quality control was executed by automatic judgement software, and it was shown that the measurements of all samples to be measured were adequately performed.

This Experimental Example 3 shows an example of measurements of 42 samples and quality control in case that the maximum sample number is 48 for one batch. The present invention is typically applicable to a case of multiple samples to be measured. However, the present invention can also be applicable to a case of a single sample to be measured by performing the start QC step, the sample measurement step, and the final QC step in this order. According to the total number of samples to be measured, the number of samples to be measured for the each sample measurement step may be adjusted. Thus, measurement and quality control can be carried out using the QC standard sample of the present invention.

As described above, in the present invention, the QC sample is prepared, wherein the QC sample contains an amyloid β peptide labeled with a stable isotope (SIL-amyloid β peptide) that is spiked into a commercially available human plasma or a pseudo plasma, and that is not originally existing in a commercially available human plasma as well as a pseudo plasma. A spike amount of the SIL-amyloid β peptide may be an amount close to an amount of the corresponding amyloid β peptide contained in the actual sample to be measured (i.e., the sample to be measured). Thus, the above-mentioned ratio of SIL-amyloid β peptide is adjusted, so that the ratio of SIL-amyloid β peptide should be within a certain definite range.

As to the prepared QC sample, by using multiple lots of antibody beads, performing measurement of multiple batches and calculating the average, the "reference value" of the SIL-amyloid β peptide ratio is determined, in the QC sample of said lot.

As to the QC sample that is subjected to measurement in the same batch at the actual sample measurement, the judgement is performed whether or not the two SIL-amyloid β peptide ratios are within the range of not less than minus (-) 20 % and not more than plus (+) 20 % with respect to the respective "reference values".

The measurement values of the sequential actual samples sandwiched between the QC samples that meet the accepted requirements are only adopted.

By controlling the amount of the SIL-amyloid β peptide in the QC sample, the QC sample close to the actual sample to be measured in composition can be prepared. By determining the reference value of the SIL-amyloid β peptide ratio in the QC sample in advance, it is possible to reduce the risk overlooking the matter that the defect of analytic system (the defect of the device condition) is gradually occurred. Furthermore, as to the QC sample measured at a certain interval, by adopting only the measurement values of the sequential actual samples sandwiched between the QC samples that meet the accepted requirements, it is possible to determine whether or not the obtained data should be adopted without overlooking a local decrease in sensitivity or the like in the batch.

The present invention includes, for example, the following aspects.
(1) A QC standard sample for measurement of a target peptide, the QC standard sample comprising:
   a blood sample comprising a peptide, and
   a peptide labeled with a stable isotope that is spiked into said blood sample.
(2) The QC standard sample according to the above (1), wherein the blood sample is selected from the group consisting of a whole blood, a plasma, and a serum.
(3) The QC standard sample according to the above (1), wherein the blood sample is selected from the group consisting of a pseudo blood, a pseudo plasma, and a pseudo serum, each of which is spiked by a peptide therein.
(4) The QC standard sample according to any one of the above (1) to (3), wherein the peptide labeled with a stable isotope that is spiked into the blood sample includes a peptide that has the same chemical structure as or an analogous chemical structure to that of the target peptide to be measured and that is labeled with a stable isotope.
(5) The QC standard sample according to any one of the above (1) to (4), wherein the target peptide to be measured includes multiple kinds of peptides,
   the peptide labeled with a stable isotope that is spiked into the blood sample includes multiple kinds of peptides that respectively have the same chemical structure as or the analogous chemical structure to that of the multiple kinds of target peptides and that are labeled with a stable isotope.
(6) The QC standard sample according to any one of the above (1) to (5), wherein the peptide labeled with a stable isotope that is spiked into the blood sample is contained in an amount within a predetermined reference range.
(7) The QC standard sample according to any one of the above (1) to (6), wherein the target peptide to be measured is an amyloid beta (Aβ) related peptide.
(8) The QC standard sample according to any one of the above (1) to (7), wherein the peptide labeled with a stable isotope that is spiked into the blood sample is an amyloid beta (Aβ) related peptide labeled with a stable isotope.
(9) The QC standard sample according to any one of the above (1) to (8), wherein the target peptide to be measured includes at least one selected from the group consisting of Aβ1-42 (SEQ ID NO.: 9), Aβ1-38 (SEQ ID NO.: 11), Aβ1-40 (SEQ ID NO.: 7), and APP669-711 (SEQ ID NO.: 10).
(10) The QC standard sample according to any one of the above (1) to (9), wherein the peptide labeled with a stable isotope that is spiked into the blood sample includes at least one selected from the group consisting of SIL-Aβ1-42, SIL-Aβ1-38, SIL-Aβ1-40, and SIL-APP669-711, wherein "SIL-" indicates "stable isotope labeled".
(11) The QC standard sample according to any one of the above (1) to (10), wherein the target peptide to be measured includes:
   Aβ1-42 (SEQ ID NO.: 9); and
   at least one selected from the group consisting of Aβ1-40 (SEQ ID NO.: 7), and APP669-711 (SEQ ID NO.: 10), and
   a ratio of measured peptide level:
      a ratio of Aβ1-40/Aβ1-42; and/or
      a ratio of APP669-711/Aβ1-42,
   is/are used as a biomarker;
   the peptide labeled with a stable isotope that is spiked into the blood sample includes:
   SIL-Aβ1-42; and
   at least one selected from the group consisting of SIL-Aβ1-40, and SIL-APP669-711, and
   a ratio of abundance of the peptide labeled with a stable isotope:
      a ratio of SIL-Aβ1-40 / SIL-Aβ1-42; and/or
      a ratio of SIL-APP669-711 / SIL-Aβ1-42,
   is/are within a predetermined reference range.
(12) The QC standard sample according to the above (11), wherein as to a ratio of abundance of the peptide labeled with a stable isotope,
   a ratio of SIL-Aβ1-40 / SIL-Aβ1-42 is within a reference range from 25.0 to 32.5; and/or
   a ratio of SIL-APP669-711 / SIL-Aβ1-42 is within a reference range from 0.7 to 1.3.
(13) A measurement method of a target peptide in multiple blood samples to be measured, the method comprising the steps of:
   a start QC step of performing a measurement of a QC standard sample that comprises a blood sample comprising a peptide, and a peptide labeled with a stable isotope that is spiked into said blood sample;
   a sample measurement step of performing a measurement of one or more blood samples to be measured;
   an intermediate QC step of performing a measurement of the QC standard sample;
   a repeating step of repeating the sample measurement step and the intermediate QC step predetermined number of times;
   a sample measurement step of performing a measurement of one or more blood samples to be measured; and
   a final QC step of performing a measurement of the QC standard sample.
(14) The measurement method of a peptide according to the above (13), wherein in each of the sample measurement steps, consecutive measurements of not less than one and not more than nine blood samples to be measured are carried out.
(15) The measurement method of a peptide according to the above (13) or (14), wherein the measurement method further comprises the steps of:
   a judgement step of deciding that if a measured level of the peptide labeled with a stable isotope in each QC standard sample in the start QC step, the intermediate QC step and the final QC step is within a normal range of not less than minus (-) 20 % and not more than plus (+) 20 % with respect to a predetermined reference value, the measurement of said QC standard sample is accepted, whereas if a measured level of the peptide labeled with a stable isotope in each QC standard sample in the start QC step, the intermediate QC step and the final QC step is out of the normal range, the measurement of said QC standard sample is rejected; and
   an adoption determination step of determining that if both measurements of the measurement of the QC standard sample performed at the QC step immediately before each sample measurement step and the measurement of the QC standard sample performed at the QC step immediately after said sample measurement step are accepted, the results of the measurement of one or more blood samples in said sample measurement step are adopted, whereas if either one or both measurements of the measurement of the QC standard sample performed at the QC step immediately before each sample measurement step and the measurement of the QC standard sample performed at the QC step immediately after said sample measurement step are rejected, the results of the measurement of one or more blood samples in said sample measurement step are not adopted.
(16) The measurement method of a peptide according to any one of the above (13) to (15), wherein the target peptide to be measured is an amyloid beta (Aβ) related peptide.
(17) The measurement method of a peptide according to any one of the above (13) to (16), wherein the peptide labeled with a stable isotope contained in the QC standard sample is an amyloid beta (Aβ) related peptide that is labeled with a stable isotope.
(18) The measurement method of a peptide according to any one of the above (13) to (17), wherein the target peptide to be measured includes at least one selected from the group consisting of Aβ1-42 (SEQ ID NO.: 9), Aβ1-38 (SEQ ID NO.: 11), Aβ1-40 (SEQ ID NO.: 7), and APP669-711 (SEQ ID NO.: 10).
(19) The measurement method of a peptide according to any one of the above (13) to (18), wherein the peptide labeled with a stable isotope contained in the QC standard sample includes at least one selected from the group consisting of SIL-Aβ1-42, SIL-Aβ1-38, SIL-Aβ1-40, and SIL-APP669-711, wherein "SIL-" indicates "stable isotope labeled".
(20) The measurement method of a peptide according to any one of the above (13) to (19), wherein the target peptide to be measured includes:
   Aβ1-42 (SEQ ID NO.: 9); and
   at least one selected from the group consisting of Aβ1-40 (SEQ ID NO.: 7), and APP669-711 (SEQ ID NO.: 10), and
   a ratio of measured peptide level:
      a ratio of Aβ1-40/Aβ1-42; and/or
      a ratio of APP669-711/Aβ1-42,
   is/are used as a biomarker;
   the peptide labeled with a stable isotope contained in the QC standard sample includes:
   SIL-Aβ1-42; and
   at least one selected from the group consisting of SIL-Aβ1-40, and SIL-APP669-711, and
   in the QC standard sample, a ratio of abundance of the peptide labeled with a stable isotope:
      a ratio of SIL-Aβ1-40 / SIL-Aβ1-42; and/or
      a ratio of SIL-APP669-711 / SIL-Aβ1-42,
   is/are within a predetermined reference range.
(21) The measurement method of a peptide according to the above (20), wherein as to a ratio of abundance of the peptide labeled with a stable isotope in the QC standard sample,
   a ratio of SIL-Aβ1-40 / SIL-Aβ1-42 is within a reference range from 25.0 to 32.5; and/or
   a ratio of SIL-APP669-711 / SIL-Aβ1-42 is within a reference range from 0.7 to 1.3.
(22) The measurement method of a peptide according to any one of the above (13) to (21), wherein the measurement is performed by using an immunoprecipitation (IP) - mass spectrometry (IP-MS), a liquid chromatography - mass spectrometry (LC-MS), or a gas chromatography - mass spectrometry (GC-MS).
(23) A QC standard sample kit for measurement of a target peptide, the QC standard sample kit comprising:
   a blood sample comprising a peptide, and
   a peptide labeled with a stable isotope that is to be spiked into said blood sample.
(24) The QC standard sample kit according to the above (23), wherein the peptide labeled with a stable isotope includes:
   SIL-Aβ1-42; and
   at least one selected from the group consisting of SIL-Aβ1-40, and SIL-APP669-711, wherein "SIL-" indicates "stable isotope labeled".

## Claims

1. A QC standard sample for measurement of a target peptide, the QC standard sample comprising:
a blood sample comprising a peptide, and
a peptide labeled with a stable isotope that is spiked into said blood sample.

2. The QC standard sample according to claim 1, wherein the blood sample is selected from the group consisting of a whole blood, a plasma, and a serum.

3. The QC standard sample according to claim 1, wherein the blood sample is selected from the group consisting of a pseudo blood, a pseudo plasma, and a pseudo serum, each of which is spiked by a peptide therein.

4. The QC standard sample according to claim 1, wherein the peptide labeled with a stable isotope that is spiked into the blood sample includes a peptide that has the same chemical structure as or an analogous chemical structure to that of the target peptide to be measured and that is labeled with a stable isotope.

5. The QC standard sample according to claim 1, wherein the target peptide to be measured includes multiple kinds of peptides, the peptide labeled with a stable isotope that is spiked into the blood sample includes multiple kinds of peptides that respectively have the same chemical structure as or the analogous chemical structure to that of the multiple kinds of target peptides and that are labeled with a stable isotope.

6. The QC standard sample according to claim 1, wherein the peptide labeled with a stable isotope that is spiked into the blood sample is contained in an amount within a predetermined reference range.

7. The QC standard sample according to claim 1, wherein the target peptide to be measured is an amyloid beta (Aβ) related peptide.

8. The QC standard sample according to claim 1, wherein the peptide labeled with a stable isotope that is spiked into the blood sample is an amyloid beta (Aβ) related peptide labeled with a stable isotope.

9. The QC standard sample according to claim 1, wherein the target peptide to be measured includes at least one selected from the group consisting of Aβ1-42 (SEQ ID NO.: 9), Aβ1-38 (SEQ ID NO.: 11), Aβ1-40 (SEQ ID NO.: 7), and APP669-711 (SEQ ID NO.: 10).

10. The QC standard sample according to claim 1, wherein the peptide labeled with a stable isotope that is spiked into the blood sample includes at least one selected from the group consisting of SIL-Aβ1-42, SIL-Aβ1-38, SIL-Aβ1-40, and SIL-APP669-711, wherein "SIL-" indicates "stable isotope labeled".

11. The QC standard sample according to claim 1, wherein the target peptide to be measured includes:
Aβ1-42 (SEQ ID NO.: 9); and
at least one selected from the group consisting of Aβ1-40 (SEQ ID NO.: 7), and APP669-711 (SEQ ID NO.: 10), and
a ratio of measured peptide level:
a ratio of Aβ1-40/Aβ1-42; and/or
a ratio of APP669-711/Aβ1-42,
is/are used as a biomarker;
the peptide labeled with a stable isotope that is spiked into the blood sample includes:
SIL-Aβ1-42; and
at least one selected from the group consisting of SIL-Aβ1-40, and SIL-APP669-711, and
a ratio of abundance of the peptide labeled with a stable isotope:
a ratio of SIL-Aβ1-40 / SIL-Aβ1-42; and/or
a ratio of SIL-APP669-711 / SIL-Aβ1-42,
is/are within a predetermined reference range.

12. The QC standard sample according to claim 11, wherein as to a ratio of abundance of the peptide labeled with a stable isotope,
a ratio of SIL-Aβ1-40 / SIL-Aβ1-42 is within a reference range from 25.0 to 32.5; and/or
a ratio of SIL-APP669-711 / SIL-Aβ1-42 is within a reference range from 0.7 to 1.3.

13. A measurement method of a target peptide in multiple blood samples to be measured, the method comprising the steps of:
a start QC step of performing a measurement of a QC standard sample that comprises a blood sample comprising a peptide, and a peptide labeled with a stable isotope that is spiked into said blood sample;
a sample measurement step of performing a measurement of one or more blood samples to be measured;
an intermediate QC step of performing a measurement of the QC standard sample;
a repeating step of repeating the sample measurement step and the intermediate QC step predetermined number of times;
a sample measurement step of performing a measurement of one or more blood samples to be measured; and
a final QC step of performing a measurement of the QC standard sample.

14. The measurement method of a peptide according to claim 13, wherein in each of the sample measurement steps, consecutive measurements of not less than one and not more than nine blood samples to be measured are carried out.

15. The measurement method of a peptide according to claim 13, wherein the measurement method further comprises the steps of:
a judgement step of deciding that if a measured level of the peptide labeled with a stable isotope in each QC standard sample in the start QC step, the intermediate QC step and the final QC step is within a normal range of not less than minus (-) 20 % and not more than plus (+) 20 % with respect to a predetermined reference value, the measurement of said QC standard sample is accepted, whereas if a measured level of the peptide labeled with a stable isotope in each QC standard sample in the start QC step, the intermediate QC step and the final QC step is out of the normal range, the measurement of said QC standard sample is rejected; and
an adoption determination step of determining that if both measurements of the measurement of the QC standard sample performed at the QC step immediately before each sample measurement step and the measurement of the QC standard sample performed at the QC step immediately after said sample measurement step are accepted, the results of the measurement of one or more blood samples in said sample measurement step are adopted, whereas if either one or both measurements of the measurement of the QC standard sample performed at the QC step immediately before each sample measurement step and the measurement of the QC standard sample performed at the QC step immediately after said sample measurement step are rejected, the results of the measurement of one or more blood samples in said sample measurement step are not adopted.

16. The measurement method of a peptide according to claim 13, wherein the target peptide to be measured is an amyloid beta (Aβ) related peptide.

17. The measurement method of a peptide according to claim 13, wherein the peptide labeled with a stable isotope contained in the QC standard sample is an amyloid beta (Aβ) related peptide that is labeled with a stable isotope.

18. The measurement method of a peptide according to claim 13, wherein the target peptide to be measured includes at least one selected from the group consisting of Aβ1-42 (SEQ ID NO.: 9), Aβ1-38 (SEQ ID NO.: 11), Aβ1-40 (SEQ ID NO.: 7), and APP669-711 (SEQ ID NO.: 10).

19. The measurement method of a peptide according to claim 13, wherein the peptide labeled with a stable isotope contained in the QC standard sample includes at least one selected from the group consisting of SIL-Aβ1-42,SIL-Aβ1-38, SIL-Aβ1-40, and SIL-APP669-711, wherein "SIL-" indicates "stable isotope labeled".

20. The measurement method of a peptide according to claim 13, wherein the target peptide to be measured includes:
Aβ1-42 (SEQ ID NO.: 9); and
at least one selected from the group consisting of Aβ1-40 (SEQ ID NO.: 7), and APP669-711 (SEQ ID NO.: 10), and
a ratio of measured peptide level:
a ratio of Aβ1-40/Aβ1-42; and/or
a ratio of APP669-711/Aβ1-42,
is/are used as a biomarker;
the peptide labeled with a stable isotope contained in the QC standard sample includes:
SIL-Aβ1-42; and
at least one selected from the group consisting of SIL-Aβ1-40, and SIL-APP669-711, and
in the QC standard sample, a ratio of abundance of the peptide labeled with a stable isotope:
a ratio of SIL-Aβ1-40 / SIL-Aβ1-42; and/or
a ratio of SIL-APP669-711 / SIL-Aβ1-42,
is/are within a predetermined reference range.

21. The measurement method of a peptide according to claim 20, wherein as to a ratio of abundance of the peptide labeled with a stable isotope in the QC standard sample,
a ratio of SIL-Aβ1-40 / SIL-Aβ1-42 is within a reference range from 25.0 to 32.5; and/or
a ratio of SIL-APP669-711 / SIL-Aβ1-42 is within a reference range from 0.7 to 1.3.

22. The measurement method of a peptide according to claim 13, wherein the measurement is performed by using an immunoprecipitation (IP) - mass spectrometry (IP-MS), a liquid chromatography - mass spectrometry (LC-MS), or a gas chromatography - mass spectrometry (GC-MS).

23. A QC standard sample kit for measurement of a target peptide, the QC standard sample kit comprising:
a blood sample comprising a peptide, and
a peptide labeled with a stable isotope that is to be spiked into said blood sample.

24. The QC standard sample kit according to claim 23, wherein the peptide labeled with a stable isotope includes:
SIL-Aβ1-42; and
at least one selected from the group consisting of SIL-Aβ1-40, and SIL-APP669-711, wherein "SIL-" indicates "stable isotope labeled".
